(19)

**Europäisches Patentamt**
**European Patent Office**
**Office européen des brevets**

(11)  **EP 4 491 734 A2**

(12)  **DEMANDE DE BREVET EUROPEEN**

(43) Date de publication:
**15.01.2025  Bulletin 2025/03**

(21) Numéro de dépôt: **24187457.7**

(22) Date de dépôt: **09.07.2024**

(51) Classification Internationale des Brevets (IPC):
$C12P\ 1/02^{(2006.01)}$          $C12P\ 1/04^{(2006.01)}$
$C12P\ 39/00^{(2006.01)}$         $A01N\ 25/02^{(2006.01)}$
$A01N\ 25/12^{(2006.01)}$         $A01N\ 63/22^{(2020.01)}$
$A01N\ 63/28^{(2020.01)}$         $A01N\ 63/32^{(2020.01)}$
$A01N\ 63/38^{(2020.01)}$         $A01P\ 21/00^{(2006.01)}$
$A23K\ 10/12^{(2016.01)}$         $A23K\ 10/16^{(2016.01)}$
$A23K\ 10/38^{(2016.01)}$         $A23K\ 30/18^{(2016.01)}$
$C05F\ 5/00^{(2006.01)}$          $C05F\ 17/00^{(2020.01)}$
$A01N\ 43/16^{(2006.01)}$         $A01P\ 1/00^{(2006.01)}$
$A01P\ 3/00^{(2006.01)}$          $A01P\ 7/04^{(2006.01)}$
$A23K\ 10/37^{(2016.01)}$

(52) Classification Coopérative des Brevets (CPC):
(C-Sets disponibles)
C12P 39/00; A01N 43/16; A01P 1/00; A01P 3/00;
A01P 7/04; A01P 21/00; A23K 10/12; A23K 10/18;
A23K 10/37; A23K 10/38; A23K 40/10;
A23K 40/25; A23K 50/10; C05F 5/002; C05F 5/008;
(Cont.)

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH MA MD TN**

(30) Priorité: **10.07.2023  FR 2307343**

(71) Demandeur: **WetterBiotech**
**64250 Itxassou (FR)**

(72) Inventeur: **Wetterwald, Paul Jean Daniel François**
**64600 Anglet (FR)**

(74) Mandataire: **Aquinov**
**12, Cours Xavier Arnozan**
**33000 Bordeaux (FR)**

(54)  **PROCÉDÉ DE TRANSFORMATION DE BIODÉCHETS ET SOUS-PRODUITS ISSUS DE L'INDUSTRIE AGROALIMENTAIRE EN BIOSOLUTIONS**

(57)  La présente invention se rapporte au domaine de l'agriculture et plus particulièrement la fabrication de biosolutions destinées à améliorer la fertilisation des cultures, la protection des cultures, ainsi que la santé animale. L'invention concerne notamment un procédé de de transformation de biodéchets et sous-produits issus de l'industrie agroalimentaire en biostimulants.

**(Cont. page suivante)**

EP 4 491 734 A2

(52) Classification Coopérative des Brevets (CPC):
(Cont.)**C05F 17/20; C12P 1/02; C12P 1/04;**
A23K 30/18

C-Sets
**A01N 63/22, A01N 25/02**

## Description

### Domaine technique

L'invention concerne la fabrication de biosolutions destinées à améliorer la fertilisation des cultures, la protection des cultures, ainsi que la santé animale. L'invention se rapporte plus particulièrement à un procédé de transformation de biodéchets et de sous-produits issus de l'industrie agroalimentaire en biosolutions pour l'agriculture et l'alimentation, notamment des produits de biocontrôle, des biostimulants, des biofertilisants, des conservateurs agroalimentaires, des conservateurs ou des produits phytosanitaires post-récolte, ainsi que des probiotiques.

### Etat de l'art

Les enjeux environnementaux sont une préoccupation majeure dans nos sociétés. Il est donc nécessaire de repenser en profondeur nos modèles économique et social afin de lutter contre le changement climatique, l'effondrement de la biodiversité, la diminution des ressources naturelles, ainsi que l'augmentation des risques environnementaux.

Dans ce contexte, la réduction de l'usage des produits phytopharmaceutiques et des intrants de synthèse ou d'origine fossile constitue une attente citoyenne forte et une nécessité pour préserver notre santé et la biodiversité. A ce titre, la plupart des gouvernements, en particulier occidentaux, prennent des mesures, notamment réglementaires pour répondre à ces attentes. En France, par exemple, le plan Ecophyto vise à réduire et/ou améliorer les usages de produits de fertilisation et phytopharmaceutiques, accélérer le retrait des substances les plus préoccupantes, renforcer la prévention de l'exposition de la population aux pesticides et leurs impacts sur l'environnement et la biodiversité, redynamiser la biodiversité des sols et les symbioses entre les plantes et les micro-organismes, source d'adaptation et de résilience face aux stress biotiques et abiotiques accélérés par le dérèglement climatique, promouvoir la reconnaissance et la diffusion des produits de biocontrôle et des préparations naturelles, mais également accompagner les agriculteurs dans cette transition.

Il existe donc un besoin pour de nouveaux produits destinés à améliorer la fertilisation des cultures, la protection des cultures et la santé animale, accélérer le remplacement des molécules de synthèse issues de la pétrochimie et des minéraux fossiles par des molécules renouvelables de la chimie verte, des micro-organismes d'intérêt agronomique ou des substances naturelles avec un moindre impact sur la qualité de l'environnement et la santé humaine. Ceci permettant de répondre plus généralement au besoin de préserver notre santé, la biodiversité, conserver nos capacités de productions agricoles, et permettre ainsi la résilience économique et environnementale de nos exploitations agricoles. Enfin, dans le contexte actuel, il existe également un besoin de développer des filières d'économie circulaire qui permettront de substituer les intrants de synthèse ou d'origine fossile par des biosolutions biosourcées et bas carbone.

### Résumé de l'invention

Pour répondre à ces besoins, l'invention propose d'utiliser des matières, considérées comme des biodéchets ou des sous-produits issus de l'industrie agroalimentaire renouvelable, largement sous-exploités à ce jour, comme matière première pour la fabrication de biosolutions, tels que des produits de biocontrôle, des produits de biostimulation, des additifs technologiques, des conservateurs biologiques d'ensilage, des additifs zootechniques, des additifs agronomiques, des biofertilisants, des conservateurs agroalimentaires, des conservateurs ou des produits phytosanitaires post-récolte, ainsi que des probiotiques.

Par l'utilisation de biodéchets ou de sous-produits issus de l'industrie agroalimentaire, une filière d'économie circulaire est créée afin de valoriser et synthétiser des molécules à haute valeur ajoutée présentes dans cette matière première, actuellement non valorisée et de les transformer, par la suite, en biosolutions pour l'agriculture et ainsi répondre au besoin de s'affranchir des molécules de synthèse connues pour leur impact sur la biodiversité et la santé. En outre, l'utilisation de biodéchets ou de sous-produits issus de l'industrie agroalimentaire permet de mettre en oeuvre un procédé bas carbone en circuit court, sans ajout de solvants pétrochimiques et de sucres fermentescibles.

Dans ce contexte, l'inventeur a développé un nouveau procédé bas carbone de transformation de cette matière première non valorisée comprenant plusieurs étapes de transformation.

Ainsi, l'invention concerne un procédé de transformation de biodéchets et sous-produits issus de l'industrie agroalimentaire en biosolutions destinées à améliorer la fertilisation et/ou la protection des cultures, ou la santé animale, ledit procédé comprenant les étapes suivantes :

a. Collecte des biodéchets,
b. Séparation physique de la fraction solide et de la fraction liquide des biodéchets collectés,
c. Récupération de la fraction liquide,
d. extraction des molécules d'intérêt, préférentiellement lesdites molécules sont présentes dans ladite fraction liquide ou le mélange comprenant la fraction liquide et solide,

e. éventuellement, fermentation comprenant l'ajout d'au moins un microorganisme exogène d'intérêt, préférentiellement au moins un microorganisme mésophile et/ou homofermentaire,

f. Stabilisation du produit obtenu à l'étape d. ou e., avantageusement du produit fermenté,

g. Eventuellement, filtration du produit stabilisé.

L'étape de séparation, notamment une séparation physique au moyen de dispositifs spécifiques sépare les différentes phases ou les différents composants des biodéchets, préférentiellement cette étape de séparation est une étape de centrifugation-décantation, celle-ci est réalisée au moyen d'un décanteur-centrifugeur. Plus préférentiellement, une étape de centrifugation-décantation en flux continue à un débit d'au plus 100 hectolitres/heure.

Préférentiellement, l'étape de séparation physique de la fraction solide et de la fraction liquide des biodéchets collectés est mise en oeuvre avant l'étape d'extraction. Toutefois, selon une variante de l'invention, ladite l'étape de séparation physique de la fraction solide et de la fraction liquide des biodéchets collectés peut être mise en oeuvre pendant ou après l'étape d'extraction.

Selon un autre objet de l'invention, l'étape d'extraction des molécules d'intérêt se réalise avantageusement par chimie verte, c'est-à-dire sans utilisation de solvants pétrochimiques, ni synthèse de substances dangereuses. Très avantageusement, l'étape d'extraction comprend les sous-étapes suivantes : macération à froid avec solvant alcoolique issu de la fermentation naturelle, suivie d'une décoction, préférentiellement une décoction à une température d'au plus 80°C.

Selon un autre objet préféré, l'étape d'extraction est suivie d'une étape de fermentation, celle-ci utilise les carbones fermentescibles contenus dans la fraction liquide pour propager lesdits microorganismes exogènes générant eux-mêmes des métabolites organiques d'intérêt. Lesdits microorganismes exogènes sont avantageusement ajoutés dans la fraction liquide. Selon une variante, lorsque l'étape de séparation est réalisée après l'étape d'extraction, éventuellement suivi de l'étape de fermentation, lesdits microorganismes exogènes sont ajoutés directement dans le mélange.

Selon une autre variante, lorsque la fraction solide est récupérée et constitue la fraction d'intérêt, lesdits microorganismes exogènes peuvent être ajoutés dans la fraction solide.

Selon un autre objet de l'invention, l'étape d'extraction consiste en une étape de fermentation, comprenant l'ajout d'au moins un microorganisme exogène d'intérêt.

La présente étape de fermentation ne comprend pas d'apport de sucres exogènes ou de sucres alimentaires (par exemple sucres de betteraves, canne à sucre, etc.) comme carbones fermentescibles. Avantageusement, cette étape est une étape de fermentation des sucres simples et complexes issus des biodéchets sourcés en circuit court par les microorganismes en conditions mésophiles permettant un procédé dit bas carbone.

Préférentiellement, le procédé selon l'invention est ainsi bas carbone, peu consommateur d'énergie et de ressources extérieures autre que les biodéchets ou sous-produits issus de l'industrie agroalimentaire.

Préférentiellement, pour augmenter le rendement, la fermentation est réalisée à une température comprise entre 17 et 37°C et/ou un pH compris entre 3 et 7, plus préférentiellement entre 5 et 6.

L'étape de fermentation permet, avantageusement, de convertir les biodéchets ou les sous-produits agroalimentaires en de nouveaux produits dit de biosolutions que ce soit un produit de biocontrôle, un biostimulant, un biofertilisant ou un probiotique contenant leurs propres principes actifs. Pour ce faire, l'inventeur a avantageusement isolé et sélectionné des souches de microorganismes exogènes avec des capacités de fermentation en adéquation avec le milieu de culture, c'est-à-dire la fraction liquide d'une part, la fraction solide d'autre part. Selon un mode de réalisation particulier, la fraction liquide est capable de produire des métabolites d'intérêt en fonction du produit final d'intérêt.

Dans le contexte de l'invention, le microorganisme exogène d'intérêt est préférentiellement choisi parmi un ferment lactique, une bactérie d'un genre choisi parmi *Bacillus, Lactobacillus, Pseudomonas, Acetobacter, Nitrobacter, Nitrosomonas, Rhizobium, Agrobacterium,* et *Streptomyces* ; un champignon du genre *Trichoderma,* une levure du genre *Saccharomyces,* ou *Aureobasidium,* et leurs combinaisons.

Selon un autre objet de l'invention, ledit procédé comprend, selon une variante, une étape supplémentaire de décoction de la fraction liquide obtenue après l'étape d'extraction, éventuellement suivi de l'étape de fermentation.

Selon un objet préféré, le produit obtenu après l'étape d'extraction ou de fermentation est stabilisé. Ainsi, la stabilisation du produit extrait et/ou fermenté est avantageusement réalisée à chaud par traitement thermique ; ou à froid par microfiltration, par filtration UV, ou traitement acide. Plus préférentiellement, le traitement thermique est une stabilisation à chaud entre 75 et 80°C, ou le traitement acide est un dosage choisi parmi l'acide acétique, peracétique, propionique, et lactique. La stabilisation par microfiltration, par filtration UV, par traitement acide, permet notamment d'inactiver ou d'éliminer les micro-organismes dans les produits stabilisés, stoppant ainsi la réaction de fermentation. Une telle étape de stabilisation permet ainsi de répondre au cahier des charges réglementaire sans micro-organisme vivant, par exemple, les cahiers des charges Préparation Naturelle Peu Préoccupante, Substance Naturelle à Usage Biostimulant.

Selon un objet préféré de l'invention, la matière première, c'est-à-dire les biodéchets ou les sous-produits issus de l'industrie agroalimentaires, est choisie parmi les sous-produits brassicoles, les sous-produits cidricoles, les sous-produits vinicoles, les sous-produits oléicoles, les sous-produits de distillerie, les sous-produits du cacao, les sous-produits du café, les sous-produits d'amidonneries, les sous-produits des sucreries, les sous-produits des pressoirs de jus de fruit, et les sous-produits de l'industrie laitière et fromagère, et leurs combinaisons.

Selon un objet, les sous-produits brassicoles sont choisis parmi la drêche de houblon du brassage, la levure de fermentation, la purge de houblon de fermentation ou garde, la drêche de malt, les eaux résiduaires de brasserie, de distillerie et leur mélange.

Selon un objet, les sous-produits vinicoles sont choisis parmi la vinasse, la lie, les pépins de raisin, et leur mélange.

Selon un objet, les sous-produits de distillerie sont choisis parmi l'eau résiduaire de distillation, la levure de fermentation, la drêche de malt et leur mélange.

Selon un objet, les sous-produits cidricoles sont choisis parmi le marc de pomme, la lie, et leur mélange.

Selon un objet, les sous-produits oléicoles sont choisis parmi la margine, le grignon, et leur mélange.

Selon un objet, les sous-produits du cacao sont choisis parmi la liqueur de cacao, le tourteau de cacao, et leur mélange.

Selon un objet, le sous-produit du café est le marc de café.

Selon un objet, les sous-produits d'amidonnerie sont choisis parmi les pelures, les eaux résiduaires, et leur mélange.

Selon un objet, les sous-produits des sucreries sont choisis parmi la mélasse, la vinasse, et leur mélange.

Selon un objet, les sous-produits des jus de fruits sont choisis parmi le marc, les écorces d'agrumes, et leur mélange.

Selon un autre objet préféré de l'invention, le produit final, c'est-à-dire le produit transformé obtenu selon l'un des modes de réalisation du procédé selon la présente invention est un produit de biosolution choisi parmi les produits de biocontrôle, les produits de biostimulation, les additifs technologiques, les conservateurs biologiques d'ensilage, les additifs zootechniques, les additifs agronomiques, les biofertilisants, les conservateurs agroalimentaires, les conservateurs ou produits phytosanitaires post-récolte, et les probiotiques.

Selon un autre objet particulièrement préféré de l'invention, l'inventeur a identifié des couples ou des complexes spécifiques (biodéchets/microorganismes). La combinaison et la complémentarité des microorganismes et de la matière première sélectionnés renforcent son efficacité et le rendement de fabrication du produit final de biosolutions.

Ainsi, selon un mode de réalisation particulier de l'invention, la biosolution est un produit de biostimulation ou un biofertilisant et le microorganisme exogène d'intérêt est une bactérie appartenant au genre choisi parmi *Lactobacillus, Bacillus, Trichoderma, Saccharomyces, Acetobacter,* et leurs combinaisons.

Selon un autre mode de réalisation particulier de l'invention, la biosolution est un produit de biocontrôle et le microorganisme est choisi parmi *Bacillus, Trichoderma, Streptomyces, Pseudomonas, Aureobasidium, Saccharomyces,* et leurs combinaisons.

Selon un autre mode de réalisation particulier de l'invention, la biosolution est un additif zootechnique ou un probiotique et le microorganisme exogène d'intérêt est choisi parmi *Bacillus, Lactobacillus, Saccharomyces* et leurs combinaisons.

Selon un autre mode de réalisation particulier de l'invention, la biosolution est un additif agroalimentaire, ou un conservateur d'ensilage, ou un conservateur alimentaire et le microorganisme exogène d'intérêt est choisi parmi *Bacillus, Lactobacillus, Saccharomyces* et leurs combinaisons.

D'autres caractéristiques et avantages ressortiront de la description détaillée de l'invention, des exemples et des figures qui vont suivre.

## Brève description des Figures

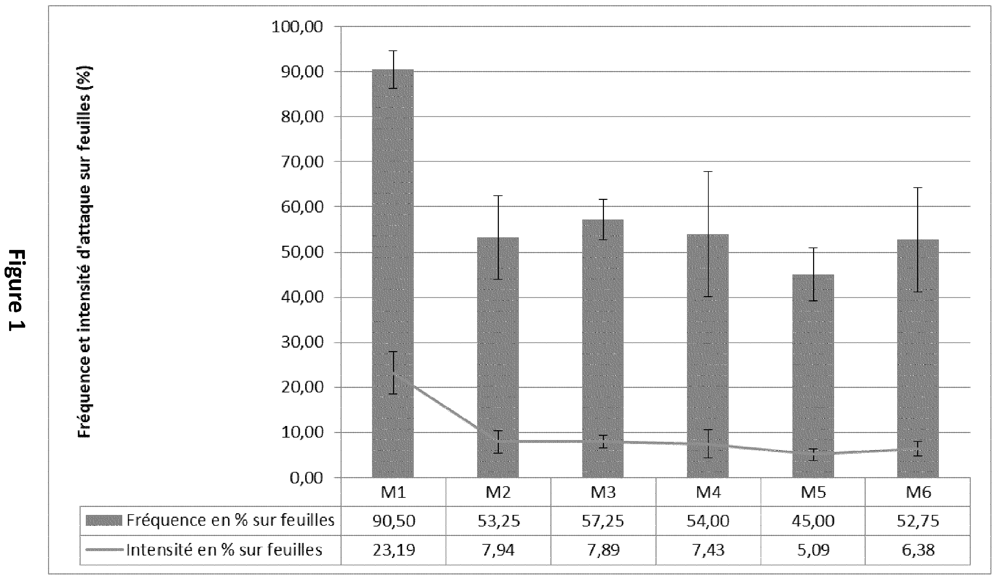

La Figure 1 représente les résultats d'efficacité de trois biosolutions selon l'invention contre le mildiou de la vigne.

## Description détaillée de l'invention

### Définition

Par « biosolutions » au sens de l'invention, on entend un ensemble de produits permettant aux agriculteurs de produire. Les biosolutions rassemblent notamment les biofertilisants, permettant aux cultures de mieux absorber les nutriments en préservant les ressources du sol grâce à la rhizosphère ; les biostimulants, stimulant les défenses de la plante vis-à-vis des stress abiotiques; les biocontrôles, imitant la nature pour protéger les plantes contre les stress biotiques ; et les additifs technologiques ou complexes de micro-organismes, favorisant la transformation de la matière organique d'un stade instable vers un stade plus stable permettant d'assurer sa meilleure conservation ou une qualité permettant de répondre à une norme pour un usage défini. Ces produits de type biosolutions peuvent se présenter sous forme solide ou liquide.

Par « biodéchet » ou « sous-produits issus de l'industrie agroalimentaire » au sens de l'invention, on entend des déchets organiques nécessairement issus de l'industrie agroalimentaire, notamment issus de ressources naturelles végétales ou animales ayant la capacité d'être également putrescibles, fermentescibles, hydrolysables, ou oxydables. Préférentiellement, les biodéchets ne sont pas des déchets ménagers aptes à être compostés, ou des effluents organiques, ou domestiques, ou des eaux usées. Plus préférentiellement, ceux-ci sont des sous-produits brassicoles, les sous-produits cidricoles, les sous-produits vinicoles, les sous-produits oléicoles, les sous-produits de distillerie, les sous-produits du cacao, les sous-produits du café, les sous-produits d'amidonneries, les sous-produits des sucreries, les sous-produits des pressoirs de jus de fruit, et les sous-produits de l'industrie laitière et fromagère.

Par « complexe » ou « couple » au sens de l'invention, on entend la combinaison et la complémentarité de microorganismes spécifiques et sélectionnés avec une matière première spécifiquement sélectionnée afin de valoriser les molécules des biodéchets, sous-produits agroalimentaires en différents métabolites d'intérêts agronomiques par voie de fermentation, hydrolyse, oxydative, avantageusement, de façon synergique entre les métabolites produits par les micro-organismes lors de cette transformation et les principes actifs végétaux contenus dans le même biodéchets ou sous-produit ayant servi de milieu de culture. Un tel complexe permettant d'obtenir un produit de biosolutions d'intérêt. A titre d'exemple, le couple Bacillus et marc de pomme pour obtenir un produit de biocontrôle.

Procédé de transformation

La présente invention a donc pour objet un procédé de transformation de biodéchets et sous-produits issus de l'industrie agroalimentaire en biosolutions destinées à améliorer la fertilisation et/ou la protection des cultures, ou la santé animale, ledit procédé comprenant au moins les étapes suivantes :

    a. Collecte des biodéchets
    b. Séparation physique de la fraction solide et de la fraction liquide des biodéchets collectés,
    c. Récupération de la fraction liquide,
    d. Extraction des molécules d'intérêt présentes dans ladite fraction liquide,
    e. Eventuellement, fermentation comprenant l'ajout d'au moins un microorganisme exogène d'intérêt,
    f. Stabilisation du produit fermenté à l'étape précédente,
    g. Eventuellement filtration du produit stabilisé.

L'étape de collecte vise à sélectionner spécifiquement des sous-produits non valorisés et riches en molécules d'intérêts, lesdites molécules d'intérêt étant préférentiellement des carbones fermentescibles, des polyphénols, des protéines, des peptides, des lipides, des bêta glucanes, des acides amers alpha, bêta, des acides organiques et des minéraux.

Selon un mode de réalisation particulier, la collecte vise à sélectionner des sous-produits brassicoles solides, liquides ou semi-liquides issus du brassage (par exemple, les drêches, purges du brassage), issus de la fermentation, (par exemple les levures, houblon purgé, purges semi-liquides de terres de filtration et levures). Ces sous-produits sont notamment riches en sucres, dextrines, amidon, oligoéléments, polyphénols (par exemple xanthohumol, catéchines, acide férulique), acides amers (lupulone), protéines, peptides, bêtaglucanes, minéraux.

Selon un autre mode de réalisation, la collecte vise à sélectionner des sous-produits cidricoles et issus des jus de pomme tels que le marc de pomme, riche en pectines, sucres et polyphénols (procyanidine, épicatéchine).

Selon un autre mode de réalisation, la collecte vise à sélectionner des sous-produits oléicoles issus des moulins à huile, particulièrement de l'huile d'olive : les margines et grignons d'olives, riches en pulpe, paroi végétale, polyphénols (hydroxytyrosol).

Selon un autre mode de réalisation, la collecte vise à sélectionner des sous-produits vinicoles issus des chais ou distilleries : les pépins et rafles de raisin riches en polyphénols (resvératrol).

Selon un autre mode de réalisation, la collecte vise à sélectionner des sous-produits de l'industrie du chocolat, particulièrement les cabosses de cacao riches en polyphénols.

Selon un autre mode de réalisation, la collecte vise à sélectionner des sous-produits d'amidonnerie ou industrie de la pomme de terre, telles que les eaux et pelures de légumes riches en amidon.

Selon un autre mode de réalisation, la collecte vise à sélectionner des sous-produits de pressoirs de jus de fruit, particulièrement les écorces d'oranges riches en polyphénols et huile essentielle.

Selon un autre mode de réalisation, la collecte vise à sélectionner des sous-produits de l'industrie laitière et fromagère, particulièrement le lactosérum (ou petit lait) riche en ferments lactiques et protéines (caséine).

Ainsi, dans le contexte de l'invention, le procédé peut être mis en oeuvre à partir d'un sous-produit relativement sous exploité ou non valorisé, mais également à partir d'un mélange de sous-produits non valorisés.

Préférentiellement, les biodéchets sont choisis parmi les sous-produits brassicoles, les sous-produits cidricoles, les sous-produits vinicoles, les sous-produits oléicoles, les sous-produits de distillerie, les sous-produits du cacao, les sous-produits du café, les sous-produits d'amidonneries, les sous-produits des sucreries, les sous-produits des pressoirs de jus de fruit, et les sous-produits de l'industrie laitière et fromagère, et leurs combinaisons.

De façon encore plus préférée, les sous-produits collectés sont choisis parmi :

- les sous-produits brassicoles sont choisis parmi le trub (purge du brassage après ébullition et décantation), la levure de fermentation, la purge de houblon, la drêche de malt, les eaux résiduaires de distillerie et leur mélange,
- les sous-produits vinicoles sont choisis parmi la vinasse, la lie, les pépins de raisin, et leur mélange,
- les sous-produits de distillerie sont choisis parmi le trub, la levure de fermentation, la drêche de malt et leur mélange,
- les sous-produits cidricoles sont choisis parmi le marc de pomme, la lie, et leur mélange,
- les sous-produits oléicoles sont choisis parmi la margine, le grignon, et leur mélange,
- les sous-produits du cacao sont choisis parmi la liqueur de cacao, le tourteau de cacao, et leur mélange,
- le sous-produit du café est le marc de café,
- les sous-produits d'amidonnerie sont choisis parmi les pelures, les eaux résiduaires, et leur mélange,
- les sous-produits des sucreries sont choisis parmi la mélasse, la vinasse, et leur mélange,
- les sous-produits des jus de fruits sont choisis parmi le marc, les écorces d'agrumes, et leur mélange ; et
- leur combinaison.

Ainsi, selon une variante, le procédé peut être mis en oeuvre à partir d'un mélange de sous-produits, par exemple des sous-produits brassicoles et oléicoles ou des sous-produits vinicoles et brassicoles, ou des sous-produits vinicoles, oléicoles et cidricoles.

Selon ce mode de réalisation, le procédé selon l'invention comprend préférentiellement une étape de collecte des sous-produits d'intérêt directement suivi d'une étape de mélange des sous-produits spécifiquement sélectionnés à l'étape précédente, c'est-à-dire avant l'étape de séparation physique. Selon une autre variante, l'étape de mélange des sous-produits spécifiquement sélectionnés peut être mise en oeuvre ultérieurement, avantageusement, après l'étape de séparation physique.

Le procédé selon l'invention comprend donc, selon un objet, une étape de séparation, celle-ci vise à séparer les différentes phases ou différents composants des biodéchets. Avantageusement, cette étape vise à obtenir une fraction liquide et une fraction solide. Très avantageusement, cette étape de séparation peut être réalisée avant, pendant, ou après l'étape d'extraction des molécules d'intérêt, ladite étape étant éventuellement suivie d'une étape de fermentation.

Avantageusement, les produits de biosolutions, notamment les produits de biocontrôle et de biostimulation sont destinés à être pulvérisés sous forme liquide, il est donc nécessaire de séparer les composants solides du liquide afin de ne pas laisser de particules en suspension qui pourraient boucher les buses du pulvérisateur et affecter la qualité de l'application du produit et par conséquent son efficacité. Préférentiellement, la fraction liquide est donc récupérée.

Selon une variante, la fraction solide peut également être récupérée, afin de développer des produits sous forme solide.

Aussi, pour les sous-produits destinés à être fermentés, la phase solide est séparée de la phase liquide, préférentiellement, avant l'étape de fermentation. Selon une variante, pour certains sous-produits destinés à subir une étape d'extraction des molécules d'intérêt sans fermentation, la phase solide est avantageusement séparée de la phase liquide après l'étape d'extraction.

Préférentiellement, lorsque le procédé comprend uniquement une étape de fermentation, c'est-à-dire lorsque que l'étape d'extraction consiste en une étape de fermentation afin d'extraire les molécules d'intérêt, celui-ci comprend une étape de séparation de la fraction solide et de la fraction liquide, puis une récupération de la fraction liquide, enfin, l'étape de fermentation est mise en oeuvre. Alternativement, la fraction solide est récupérée, puis l'étape de fermentation est mise en oeuvre.

Selon une autre variante préféré, lorsque le procédé comprend uniquement une étape d'extraction, celui-ci comprend l'étape d'extraction suivie d'une étape de séparation de la fraction solide et liquide et récupération de la fraction liquide.

Selon une autre variante, le procédé peut comprendre une étape d'extraction suivie d'une étape de fermentation.

Selon un autre objet particulièrement préféré, l'étape de séparation est réalisée au moyen d'un décanteur-centrifugeur. Son principe de fonctionnement est avantageux dans le contexte de l'invention. En effet, la centrifugation est mise en oeuvre avec un axe de rotation horizontal et non vertical permettant d'atteindre un taux de matière sèche assez élevé et donc d'optimiser la séparation en limitant les pertes d'efficacité de la séparation solide/liquide. A titre d'exemple, une purge de levures de bière récoltée à 20% de matière sèche sort du décanteur-centrifugeuse avec 85% de matière sèche.

Une fois l'étape de séparation terminée, et donc la fraction liquide séparée de la fraction solide (matière sèche), ladite fraction liquide comprend des sucres fermentescibles, des polyphénols et d'autres molécules d'intérêt utilisées pour l'étape d'extraction et fermentation pour obtenir les produits de biosolution, notamment des produits de biocontrôle et biostimulation.

Selon un autre objet, le solide séparé comportant de l'amidon, de la cellulose, des levures en fonction du type de sous-produit pourra être utilisé dans un procédé de fabrication d'aliments et compléments alimentaires type probiotiques pour la nutrition et santé animale. A titre d'exemple, la levure de bière concentrée au moyen du décanteur-centrifugeur à 85% de matière sèche pourra être conditionnée au moyen d'une machine de presse à granules ou une extrudeuse. Selon un mode de réalisation préféré, le solide fermentescible pourra être ensemencé avec un micro-organisme d'intérêt probiotique pour la santé animale (par exemple une levure, un lactobacille ou un bacille), fermenté et conditionné après séchage via une presse à granules ou une extrudeuse.

A titre d'exemple, lorsque les probiotiques solides sont destinés à l'alimentation animale (levure de bière, complément probiotique à lactobacilles), le produit est transformé en granulés au moyen d'une extrudeuse permettant d'améliorer la durée de conservation (taux d'humidité réduit suite à l'extrusion).

Lorsque la fraction liquide est séparée et récupérée, une étape d'extraction des molécules d'intérêt présentes dans ladite fraction liquide est mise en oeuvre et/ou une étape de fermentation.

Préférentiellement, l'extraction des molécules d'intérêt est une extraction par chimie verte c'est-à-dire dépourvue de substances toxiques, par exemple des solvants pétrochimiques, ou connues pour avoir des effets indésirables et plus généralement un impact sur l'environnement et la santé humaine. Ainsi, l'extraction est préférentiellement mise en oeuvre au moyen de substances naturelles avec un faible impact sur la qualité de l'environnement et la santé humaine.

Selon un objet préféré de l'invention, l'étape d'extraction comprend les sous-étapes suivantes :

a. macération à froid avec solvant alcoolique issu de la fermentation naturelle,

b. décoction, et

c. éventuellement, une étape d'extraction sous vide ou par ultrason ou micro-onde.

Ainsi, la macération avec un alcool naturellement présent dans le sous-produit permet l'extraction des molécules organiques d'intérêt. Enfin, la décoction, apportant de la chaleur, permet d'accélérer les réactions, notamment l'isomérisation des molécules d'intérêt, augmentant ainsi l'efficacité de la biosolution.

Selon une variante de l'invention, l'extraction peut comprendre en outre une étape d'extraction mise en oeuvre par ultrason, micro-onde, sous vide ou sous azote afin d'augmenter encore les rendements d'extraction, d'extraire les molécules sensibles à l'oxydation et ainsi limiter le phénomène d'oxydation.

Selon un exemple particulier, lorsque le procédé vise à transformer un sous-produit brassicole en produit de biostimulation, le sous-produit liquide est chauffé lors d'une étape de décoction à 80°C avec agitation et incorporation des purges de houblons. Les polyphénols et acides amers du houblon isomérisent et se dissolvent dans la décoction. Ce procédé est alors catalysé par la chaleur et la présence d'alcool et dure au minimum 3 heures afin d'évaporer au fur et à mesure l'alcool et aboutir à une concentration d'alcool quasi nulle, proche de zéro. En outre, ce procédé présente avantageusement, comme effet indirect, la stérilisation de la fraction liquide et sa concentration en acides amers, polyphénols et molécules d'intérêt.

Dans le contexte de l'invention, en remplacement de l'étape d'extraction ou lorsque l'étape d'extraction est terminée, le procédé comprend une étape de fermentation comprenant l'ajout d'au moins un microorganisme exogène d'intérêt afin d'utiliser les carbones fermentescibles contenus dans la fraction liquide pour propager lesdits microorganismes exogènes générant eux-mêmes des métabolites organiques d'intérêt.

Les conditions de fermentation dépendent du microorganisme utilisé. Ainsi, l'homme du métier à partir de ses connaissances générales peut adapter les conditions de fermentation en fonction du microorganisme sélectionné. A titre d'exemple, un *Bacillus subtilis* est une bactérie anaérobie facultative et nécessite des conditions particulières pour sa bonne sporulation. Les conditions de sporulation du genre de bactérie *Bacillus* sont une diminution de la biodisponibilité de nutriments et des conditions de température et pH moins favorables à sa survie (en-dessous de 15°C, au-dessus de 40°C, pH < 2, pH > 8).

Selon un autre exemple, un *Lactobacillus plantarum* doit être cultivé en anaérobiose et se conserve moins bien car il ne forme pas de spores.

Par conséquent, les paramètres clefs de la fermentation sont suivis, notamment le pH, la température, la croissance microbienne, population, viabilité et la concentration des métabolites d'intérêt tels que l'acide lactiques, enzymes, hormones de croissance, lipopeptide, molécules surfactantes etc.

Selon un objet préféré de l'invention, pour améliorer le rendement, la fermentation est réalisée à une température comprise entre 17 et 37°C et/ou un pH compris entre 3 et 7, plus préférentiellement entre 5 et 6.

L'étape de fermentation permet, avantageusement, de convertir les biodéchets ou sous-produits agroalimentaires en de nouveaux produits de biosolutions que ce soit un biocontrôle, un biostimulant, un biofertilisant ou un probiotique contenant leurs propres principes actifs. Pour ce faire, l'inventeur a avantageusement isolé et sélectionné des souches de microorganismes exogènes avec des capacités de fermentation en adéquation avec le milieu de culture, c'est-à-dire la fraction liquide ou la fraction solide, et capables de produire des métabolites d'intérêt en fonction du produit final d'intérêt.

Pour y parvenir, le microorganisme exogène d'intérêt est préférentiellement choisi parmi un ferment lactique, une bactérie d'un genre choisi parmi *Bacillus, Lactobacillus, Pseudomonas, Acetobacter, Nitrobacter, Nitrosomonas, Rhizobium, Agrobacterium,* et *Streptomyces* ; un champignon du genre *Trichoderma,* une levure du genre *Saccharomyces,* ou *Aureobasidium,* et leurs combinaisons.

Plus préférentiellement, l'inventeur s'est intéressé, sans être limitatif, aux bactéries *Bacillus subtilis, Bacillus amyloliquefaciens, Bacillus velezensis, Bacillus pumilus* pour leur capacité à produire des lipopeptides du type fengycine, iturine, surfactine, lichenysine ainsi qu'aux *Trichoderma* (*Trichoderma harzianum, Trichoderma viride*) et levures (règne des champignons) pour leur capacité à sécréter des toxines contre des agents fongiques pathogènes des plantes et initier une compétition pour les nutriments du milieu à coloniser.

Pour les souches destinées à la fabrication de biostimulants et activateurs de composts, fumiers, lisiers, l'inventeur s'est notamment intéressé, sans être limitatif, aux ferments lactiques, notamment les espèces : *Lactobacillus plantarum,* Lactobacillus *acidophilus, Lactobacillus helveticus, Lactococcus lactis, Propionibacterium freudenreichii,* les levures *Saccharomyces cerevisiae* et des bactéries nitrifiantes comme les *Nitrosomonas* et *Nitrobacter.*

Lorsque les biodéchets, effluents organiques ou sous-produits agroalimentaires sont riches en sucres simples et sucres complexes, ceux-ci sont fermentés préférentiellement par des levures *Saccharomyces* ou des ferments lactiques *Bacillus, Lactobacillus.*

Lorsque les biodéchets, effluents organiques ou sous-produits agroalimentaires contiennent de l'alcool, ceux-ci sont préférentiellement fermentés par des bactéries acétiques *Acetobacter.*

Lorsque les biodéchets ou sous-produits agroalimentaires sont de composition majoritaire solide (faible taux d'humidité) et riches en cellulose, ceux-ci sont transformés préférentiellement par des champignons *Trichoderma, Aureobasidium.*

Lorsque les biodéchets, effluents organiques ou sous-produits agroalimentaires sont riches en polyphénols, acides organiques difficilement fermentescibles, ceux-ci sont préférentiellement transformés par des bactéries *Streptomyces* (hydrolyse), *Pseudomonas* (voie oxydative).

Plus préférentiellement, les couples biodéchets et microorganismes sont décrits dans le tableau 1 ci-après.

[Tableau 1]

| Industrie | Sous-produit/ biodéchet | pH initial | Microorganisme exogène |
|---|---|---|---|
| brassicole | Trub/drêche de houblon | 5-6 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter: Acetobacter aceti; Trichoderma : Trichoderma harzianum, Trichoderma viride* |
| brassicole | levure fermentation | 4-5 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |
| brassicole | purge houblon fermentation | 4-5 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillusacidophilus, Lactobacillus helveticus ; Lactococcus lactis ; Saccharomyces cerevisiae; Acetobacter : Acetobacter aceti; Trichoderma : Trichoderma harzianum, Trichoderma viride* |

(suite)

| Industrie | Sous-produit/ biodéchet | pH initial | Microorganisme exogène |
|---|---|---|---|
| brassicole | drêche de malt | 5-6 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |
| vinicole | vinasse | 3-4 | *Streptomyces : Streptomyces violatus, Pseudomonas : Pseudomonas fluorescens, Nitrosomonas : Nitrosomonas europaea Nitrobacter : Nitrobacter winogradskyi, Nitrobacter hamburgensis, Rhizobium : Bradyrhizobium japonicum, Rhizobium leguminosarum, ; Agrobacterium : Agrobacterium tumefaciens, Aureobasidium : Aureobasidium pullulons* |
| vinicole | lie (levure fermentation) | 4-5 | *Bacillus, Lactobacillus, Saccharomyces, Acetobacter, Trichoderma* |
| vinicole | pépins de raisin | 5-6 | *Streptomyces : Streptomyces violatus, Pseudomonas : Pseudomonas fluorescens, Nitrosomonas : Nitrosomonas europaea Nitrobacter : Nitrobacter winogradskyi, Nitrobacter hamburgensis, Rhizobium : Bradyrhizobium japonicum, Rhizobium leguminosarum, ; Agrobacterium : Agrobacterium tumefaciens, Aureobasidium : Aureobasidium pullulons* |
| distillerie | trub | 5-6 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |
| distillerie | levure fermentation | 5-6 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |
| distillerie | drêche de malt | 5-6 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |
| cidricole | marc de pomme | 4-5 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |
| cidricole | lie (levure fermentation) | 4-5 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |

(suite)

| Industrie | Sous-produit/ biodéchet | pH initial | Microorganisme exogène |
|---|---|---|---|
| oléicole | margine | 4-5 | *Streptomyces : Streptomyces violatus, Pseudomonas : Pseudomonas fluorescens, Nitrosomonas : Nitrosomonas europaea Nitrobacter : Nitrobacter winogradskyi, Nitrobacter hamburgensis, Rhizobium : Bradyrhizobium japonicum, Rhizobium leguminosarum, ; Agrobacterium : Agrobacterium tumefaciens, Aureobasidium : Aureobasidium pullulons* |
| oléicole | grignon | 4-5 | *Streptomyces : Streptomyces violatus, Pseudomonas : Pseudomonas fluorescens, Nitrosomonas : Nitrosomonas europaea Nitrobacter : Nitrobacter winogradskyi, Nitrobacter hamburgensis, Rhizobium : Bradyrhizobium japonicum, Rhizobium leguminosarum, ; Agrobacterium : Agrobacterium tumefaciens, Aureobasidium : Aureobasidium pullulons* |
| cacao | liqueur de cacao | 5-6 | *Streptomyces : Streptomyces violatus, Pseudomonas : Pseudomonas fluorescens, Nitrosomonas : Nitrosomonas europaea Nitrobacter : Nitrobacter winogradskyi, Nitrobacter hamburgensis, Rhizobium : Bradyrhizobium japonicum, Rhizobium leguminosarum, ; Agrobacterium : Agrobacterium tumefaciens, Aureobasidium : Aureobasidium pullulons* |
| cacao | tourteau de cacao | 5-6 | *Streptomyces : Streptomyces violatus, Pseudomonas : Pseudomonas fluorescens, Nitrosomonas : Nitrosomonas europaea Nitrobacter : Nitrobacter winogradskyi, Nitrobacter hamburgensis, Rhizobium : Bradyrhizobium japonicum, Rhizobium leguminosarum, ; Agrobacterium : Agrobacterium tumefaciens, Aureobasidium : Aureobasidium pullulons* |
| café | marc de café | 6-7 | *Streptomyces : Streptomyces violatus, Pseudomonas : Pseudomonas fluorescens, Nitrosomonas : Nitrosomonas europaea Nitrobacter : Nitrobacter winogradskyi, Nitrobacter hamburgensis, Rhizobium : Bradyrhizobium japonicum, Rhizobium leguminosarum, ; Agrobacterium : Agrobacterium tumefaciens, Aureobasidium : Aureobasidium pullulons* |
| pomme de terre | pelures | 6-7 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |
| pomme de terre | eaux résiduaires | 6-7 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |
| jus de fruits | écorces d'agrumes | 5-6 | *Streptomyces : Streptomyces violatus, Pseudomonas : Pseudomonas fluorescens, Nitrosomonas : Nitrosomonas europaea Nitrobacter : Nitrobacter winogradskyi, Nitrobacter hamburgensis, Rhizobium : Bradyrhizobium japonicum, Rhizobium leguminosarum, ; Agrobacterium : Agrobacterium tumefaciens, Aureobasidium : Aureobasidium pullulons* |
| jus de fruits | marc | 5-6 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |

(suite)

| Industrie | Sous-produit/ biodéchet | pH initial | Microorganisme exogène |
|---|---|---|---|
| betterave | mélasse | 6-7 | *Bacillus : Bacillus subtilis, Bacillus amyloliquefaciens, Bacillus velezensis, Bacillus pumilus, Bacillus methylotrophicus, Paenibacillus polymyxa; Lactobacillus : Lactobacillus plantarum, Lactobacillus acidophilus, Lactobacillus helveticus ; Lactococcus lactis ; Saccharomyces cerevisiae; Acetobacter : Acetobacter aceti; Trichoderma : Trichoderma harzianum, Trichoderma viride* |
| betterave | vinasse | 5-6 | *Streptomyces : Streptomyces violatus, Pseudomonas : Pseudomonas fluorescens, Nitrosomonas : Nitrosomonas europaea Nitrobacter : Nitrobacter winogradskyi, Nitrobacter hamburgensis, Rhizobium : Bradyrhizobium japonicum, Rhizobium leguminosarum, ; Agrobacterium : Agrobacterium tumefaciens, Aureobasidium : Aureobasidium pullulons* |

Enfin, le procédé selon l'invention comprend une étape de stabilisation du produit extrait et/ou fermenté. Selon un objet préféré, l'étape de stabilisation du produit extrait et/ou fermenté est réalisée à chaud par traitement thermique ou à froid par microfiltration, filtration UV, traitement acide. Plus préférentiellement, le traitement thermique est une stabilisation à chaud entre 75 et 80°C, ou une microfiltration éventuellement complétée d'une filtration UV, éventuellement complétée par un traitement acide qui est un dosage choisi parmi l'acide acétique, peracétique, propionique, et lactique. La stabilisation permet notamment de garantir l'inactivation ou l'élimination de micro-organismes indésirables pour des raisons de conformité qualité à un cahier des charges, une norme d'autorisation de mise sur le marché et permet de garantir la durée de conservation du produit.

Une fois l'étape de stabilisation terminée, une première forme de la biosolution est obtenue. Celle-ci peut être conditionnée, par exemple en bidon de 1L, 5L, 10L, 20L, GRV de 1000L pour les produits liquides, en contenant de 1 à 25kg pour les produits solides selon le circuit de distribution et selon les connaissances de l'homme du métier.

Eventuellement, une étape de filtration peut être mise en oeuvre avant le conditionnement, notamment pour les produits liquides destinés à être pulvérisés. Pour cela le produit est filtré au moyen de décanteur-centrifugeur suivi d'une maille filtrante à 200μm afin d'assurer une séparation efficace de la fraction liquide et des éventuelles particules solides subsistant. Avantageusement, la filtration est réalisée en flux continue.

Dans le contexte de l'invention, les biosolutions sont préférentiellement choisies parmi les produits de biocontrôle, les produits de biostimulation, les additifs technologiques, les conservateurs biologiques d'ensilage, les additifs zootechniques, les additifs agronomiques, les biofertilisants, les conservateurs agroalimentaires, les conservateurs ou produits phytosanitaires post-récolte, et les probiotiques.

Les produits de biostimulation stimulent les défenses naturelles de la plante mais permettent aussi aux agriculteurs de s'affranchir de l'achat des engrais de synthèse ou d'origine fossile. En outre, ils permettent de développer la fertilisation en autarcie par le compostage des fumiers, lisiers, déchets verts, couverts végétaux, en solubilisant des nutriments liés aux composants organiques ou minéraux du complexe argilo-humique comme le phosphore et le potassium, en améliorant les processus de symbiose bactérienne et des champignons avec la plante comme la mycorhization des racines grâce à une biodiversité microbienne du sol plus étendue. Les biostimulants apportent alors des micro-organismes et des nutriments d'intérêt pour augmenter l'efficacité des processus de compostage, humification minéralisation, mycorhization, symbiose bactérienne et développement de la microflore bactérienne du sol.

Les produits de biocontrôle répondent aux enjeux environnementaux (plan Ecophyto) et visent à remplacer les molécules de synthèse issues de la pétrochimie par des molécules de la chimie verte ou des substances naturelles, par des micro-organismes secrétant des métabolites à visée phytosanitaire ou faisant la compétition pour la colonisation du milieu face aux agents pathogènes, par des macro-organismes faisant la compétition pour la colonisation du milieu face aux agents pathogènes ou prédateurs de ces mêmes agents pathogènes avec un moindre impact sur la qualité de l'environnement et la santé humaine.

Les complexes de micro-organismes sont des additifs technologiques de compostage et assainissant de litière d'élevage utilisables en agriculture biologique pour fumiers, lisiers, déchets verts, digestats. Ils contiennent des minéraux essentiels, des végétaux naturels fermentés avec des micro-organismes sélectionnés. Ils améliorent l'efficacité des engrais de ferme, digestats, composts et activent rapidement leur transformation biologique en humus. Concrètement, en acidifiant le pH et en apportant des carbones fermentescibles, ils modifient l'organisation de l'azote de la matière organique en faisant descendre le rapport C/N et réduisent fortement la volatilisation de l'azote ammoniacal ce qui améliore l'utilisation du fumier ou lisier composté par rapport au cahier des charges d'épandage. L'additif de compostage permet de nitrifier, humifier les fumiers, lisiers et diminuer les pertes d'azote ammoniacal par volatilisation et par lessivage.

Cela améliore le rendement de valorisation en fertilisation des effluents d'élevage et améliore les conditions sanitaires des élevages d'un point de vue microbiologique (moins de diarrhées, mammites, cellules pathogènes dans le lait) et troubles respiratoires liés aux odeurs d'ammoniac pour les travailleurs et les animaux d'élevage (par exemple les agneaux).

Enfin, les fumiers, lisiers, fientes compostés grâce à notre solution atteignent la norme d'amendement organique NFU44-051 autorisant la commercialisation. On passe donc d'un déchet à un produit commercial d'une valeur équivalente à un engrais de synthèse et source de bénéfice pour l'éleveur.

Les conservateurs biologiques d'ensilage sont des additifs technologiques permettant d'améliorer la fermentation lactique des fourrages lors du processus d'ensilage. Ceux-ci sont fabriqués à partir de la fermentation d'un sous-produit végétal grâce à des ferments lactiques sélectionnés du type lactobacilles homofermentaires. Il est stabilisé à pH 4,2 et contient 1 à 2% de sucres résiduels ce qui permet d'acidifier rapidement le fourrage à ensiler et d'apporter des sucres pour les fourrages pauvres comme l'herbe afin de déclencher rapidement la fermentation lactique du fourrage. Le bénéfice est une meilleure conservation des qualités nutritionnelles dans le temps et même l'amélioration des qualités nutritionnelles du fourrage ensilé ainsi que la protection contre le développement de moisissures, de clostridies et la sécrétion de mycotoxines.

Enfin, les probiotiques pour l'alimentation animale sont des compléments alimentaires contenant du vivant, c'est-à-dire des microorganismes qui vont avoir une influence sur la santé de l'animal ingérant le probiotique, essentiellement au niveau du microbiote. Certaines levures et bactéries (dans notre cas la levure de bière et des lactobacilles) améliore l'efficience de la digestion notamment chez les ruminants par leur capacité à absorber l'oxygène du rumen et favoriser ainsi le travail des bactéries anaérobies. Des études ont montré une amélioration du gain de poids moyen quotidien de 2,5 à 5% et une amélioration de l'indice de consommation de 2% (gain de poids/poids d'aliment consommé).

Selon un autre aspect, l'invention se rapporte également à des biosolutions choisis parmi les produits de biocontrôle, les produits de biostimulation, les additifs technologiques, les conservateurs biologiques d'ensilage, les additifs zootechniques, les additifs agronomiques, les biofertilisants, les conservateurs agroalimentaires, les conservateurs ou produits phytosanitaires post-récolte, et les probiotiques obtenus par le procédé selon l'un des quelconques modes de réalisation précédemment décrits.

De façon particulièrement préférée, la biosolution est un produit de biostimulation ou un biofertilisant et le microorganisme exogène d'intérêt est une bactérie appartenant au genre choisi parmi *Lactobacillus, Bacillus, Trichoderma, Saccharomyces, Acetobacter,* et leurs combinaisons.

Selon un autre mode de réalisation préféré, la biosolution est un produit de biocontrôle et le microorganisme est choisi parmi *Bacillus, Trichoderma, Streptomyces, Pseudomonas, Aureobasidium, Saccharomyces,* et leurs combinaisons.

Selon un autre mode de réalisation préféré, la biosolution est un additif zootechnique ou un probiotique et le microorganisme exogène d'intérêt est choisi parmi *Bacillus, Lactobacillus, Saccharomyces* et leurs combinaisons.

Selon un autre mode de réalisation préféré, la biosolution est un additif agroalimentaire, ou un conservateur d'ensilage, ou un conservateur alimentaire et le microorganisme exogène d'intérêt est choisi parmi *Bacillus, Lactobacillus, Saccharomyces* et leurs combinaisons.

Ainsi, le procédé selon l'invention est bas carbone, peu consommateur d'énergie et de ressources extérieures autre que les biodéchets ou sous-produits issus de l'industrie agroalimentaire et répond aux besoins de l'art antérieur.

L'invention est à présent illustrée par des exemples non limitatifs de compositions selon l'invention et par des résultats.

**Exemples**

Exemple 1 - Biostimulant de défense des plantes

Ce produit de biostimulation présente les caractéristiques suivantes : il s'agit d'un extrait de polyphénols et acides amers bêta de houblon contenant également des acides aminés, des peptides, des protéines et des oligoéléments, qui sont des molécules élicitrices permettant l'activation de gènes de défense des plantes, une activité fongifuge et bactérifuge et une activité antioxydante protectrice contre les stress oxydatifs de la plante. Il vise à être appliqué sur les parties foliaires des cultures maraîchères, la vigne, l'arboriculture, les grandes cultures (céréales, oléoprotéagineux, légumineuses, betterave), les espaces verts (gazon) et en horticulture. Ce produit est notamment obtenu selon le procédé suivant.

La purge de houblon est collectée auprès d'une usine de bière et retraitée par un procédé de décoction jusqu'à une température de 80°C pendant une durée de 3 heures afin d'extraire les polyphénols et acides amers d'intérêt, éventuellement complétée d'une extraction sous vide ou par ultrason, micro-onde, suivi d'une étape de séparation par un décanteur-centrifuge afin de récupérer la fraction liquide. Le liquide séparé est ensuite filtré à $200\mu$m. Le biostimulant est stable à un pH entre 4 et 4,5 avant son conditionnement. La concentration minimale en polyphénols calculée en équivalent acide gallique, acide tannique, catéchine ou épicatéchine est de 500mg/kg dans le biostimulant.

Exemple 2 - Biofertilisant nutritionnel

Ce biofertilisant présente les caractéristiques suivantes : il s'agit d'une solution liquide fermentée à partir de la drêche de houblon contenant des bactéries lactiques et levures et vise à être appliqué sur les semis (au niveau du sol) de grandes cultures (céréales, oléoprotéagineux, légumineuses, betterave), cultures maraichères, en vigne, arboriculture, espaces verts et horticulture.

Ce produit est notamment obtenu selon le procédé suivant.

La drêche de houblon est collectée auprès d'une usine de bière et retraitée par un procédé de séparation par décanteur-centrifuge, puis la phase liquide séparée est fermentée avec inoculation de souches sélectionnées de *Lactobacillus plantarum* et *Bacillus subtilis* dans la phase liquide. La fermentation se déroule sur une durée de 7 jours à une température de 20°C à 30°C à un pH initial de 5 à 6 et un pH final de 4 à 5. Le produit liquide de la fermentation est filtré à 200μm et stabilisé à pH 4 - 4,5 avant conditionnement. La concentration minimale en acide lactique dans le biofertilisant est de 20g/L d'acide lactique. L'acide lactique solubilise notamment le phosphore et le potassium coincé dans le complexe argilo humique.

Une variante du biofertilisant consisterait à lyophiliser les bactéries du liquide afin de conditionner le produit sous forme de poudre.

Exemple 3 - Biocontrôle fongicide

Ce produit de biocontrôle présente les caractéristiques suivantes : il s'agit de drêche de houblon fermentée grâce à l'inoculation d'une souche sélectionnée de *Bacillus subtilis* ou *Bacillus amyloliquefaciens* capable de synthétiser des molécules fongicides surfactantes (iturine, surfactine, fengycine) et vise à être appliqué sur vigne, arboriculture, grandes cultures (céréales, oléoprotéagineux, légumineuses, betterave), cultures maraîchères, espaces verts et horticulture.

Ce produit est notamment obtenu selon le procédé suivant.

La drêche de houblon est collectée auprès d'une usine de bière et retraitée par un procédé de séparation par décanteur-centrifuge, puis la phase liquide séparée est fermentée avec inoculation de souches sélectionnées de *Bacillus subtilis* ou *Bacillus amyloliquefaciens* dans la phase liquide. La fermentation se déroule sur une durée de 15 jours à une température de 20°C à 30°C à un pH initial de 5 à 6 et un pH final de 4 à 5. Le produit liquide issu de la fermentation est filtré à 200μm et stabilisé à pH 4 - 4,5 avant conditionnement.

Selon une variante les bactéries du biocontrôle sous forme liquide sont lyophilisées et le biocontrôle est alors conditionné sous forme de poudre.

Exemple 4 - Biocontrôle insectes piqueurs

Ce produit de biocontrôle présente les caractéristiques suivantes : il s'agit d'un extrait de maltodextrine (substance de base européenne efficace dans la lutte contre les insectes piqueurs suceurs) et vise à être appliqué sur la surface foliaire des cultures maraichères, de la vigne, l'arboriculture, les grandes cultures (céréales, oléoprotéagineux, légumineuses, betterave), les espaces verts et en horticulture.

Ce produit est notamment obtenu selon le procédé suivant. L'eau résiduaire maltée est collectée auprès d'une usine de bière ou une distillerie de whisky et retraitée par un procédé de décoction jusqu'à une température de 80°C afin de concentrer les sucres complexes et extraire la maltodextrine de l'amidon, suivi d'une étape de séparation par un décanteur-centrifuge afin de récupérer la fraction liquide. Le liquide séparé est ensuite filtré à 200μm. Le produit final est stabilisé à un pH entre 4 et 4,5 avant conditionnement.

Exemple 5 - Additifs technologique : complexe de micro-organismes

Cet additif technologique présente les caractéristiques suivantes : il s'agit de drêches de houblon fermentées contenant des souches sélectionnées de *Lactobacillus plantarum, Lactococcus Lactis, Bacillus subtilis, Saccharomyces cerevisiae.* Ce complexe de micro-organismes est un assainissant de litière d'élevage et activateur de fumier. Il vise à être appliqué sur litière d'élevage, tas de fumier, lisier, déchets verts afin de résoudre des problématiques sanitaires et transformer les fumiers en amendement organique commercial selon la norme NFU 44-051.

Ce produit est notamment obtenu selon le procédé suivant.

La drêche de houblon est collectée par retraitée par un procédé de séparation par décanteur-centrifuge, puis la phase liquide séparée est fermentée avec inoculation de souches sélectionnées de *Lactobacillus plantarum, Lactoccocus lactis, Bacillus subtilis, Saccharomyces cerevisiae* dans la phase liquide. La fermentation se déroule sur une durée de 15 jours à une température de 20°C à 30°C à un pH initial de 5 à 6 et un pH final de 4 à 5. Le produit liquide issu de la fermentation est filtré à 200μm et stabilisé à pH 4 - 4,5 avant conditionnement. La concentration minimale en acide lactique dans le produit final est de 20g/L d'acide lactique. Dans ce contexte, l'acide lactique permet de stopper la volatilisation de l'ammoniac et de lutter contre des bactéries pathogènes. Intérêt sanitaire et qualité de fertilisation future

Une variante du produit final consisterait à lyophiliser les bactéries du liquide afin de conditionner un produit final sous forme de poudre.

Exemple 6 - Conservateurs biologiques d'ensilage

Ce conservateur biologique d'ensilage présente les caractéristiques suivantes : il s'agit d'eau résiduaire maltée fermentée contenant une souche de *Lactobacillus plantarum* sélectionnée et vise à être appliqué sur de l'ensilage d'herbe, de l'ensilage de luzerne, de l'ensilage de maïs afin d'améliorer la fermentation lactique du processus d'ensilage et d'améliorer la conservation de l'ensilage vis-à-vis des moisissures, clostridies, mycotoxines.

Ce produit est notamment obtenu selon le procédé suivant.

L'eau résiduaire maltée est collectée auprès d'une usine de bière ou une distillerie de whisky. Elle est retraitée par un procédé de séparation par décanteur-centrifuge, puis la phase liquide séparée est fermentée avec inoculation de souches sélectionnées de *Lactobacillus plantarum* dans la phase liquide. La fermentation se déroule sur une durée de 15 jours à une température de 20°C à 30°C à un pH initial de 5 à 6 et un pH final de 4 à 5. Le produit liquide issu de la fermentation est filtré à $200 \mu m$ et stabilisé à pH 4 - 4,5 avant conditionnement. La concentration minimale en acide lactique dans le produit final est de 20g/L d'acide lactique, permettant de conserver le fourrage.

Une variante du produit final consisterait à lyophiliser les bactéries du liquide afin de conditionner un produit final sous forme de poudre.

Exemple 7 - Probiotiques ruminants

Ce probiotique présente les caractéristiques suivantes : il s'agit de drêche de houblon solide fermenté contenant des souches sélectionnées de *Lactobacillus plantarum, Bacillus subtilis, Saccharomyces cerevisiae* et vise à être utilisé en complément nutritionnel probiotique pour l'alimentation animale afin d'améliorer la performance de rumination, guérir une acidose du rumen, diminuer les diarrhées et améliorer la digestion des animaux et des Hommes en général.

Ce produit est notamment obtenu selon le procédé suivant.

De la drêche de houblon est collectée auprès d'une usine de bière puis fermenté avec inoculation de souches sélectionnées de *Lactobacillus plantarum, Bacillus subtilis, Saccharomyces cerevisiae.* La fermentation se déroule sur une durée de 3 semaines à une température de 20°C à 30°C à un pH initial de 5 à 6 et un pH final de 4 à 5. Le produit de la fermentation est retraité par un procédé de séparation par décanteur-centrifuge. La phase solide séparée est passée par une extrudeuse ou une presse à granules pour être conditionné sous forme de granulés.

**Essai d'efficacité de biosolutions selon l'invention**

Exemple 8 - Essai d'efficacité *in vitro* de biosolutions selon l'invention

Cet essai vise à évaluer l'efficacité du biocontrôle selon l'invention avec *B. subtilis* vis-à-vis de l'agent du black-rot (*Phyllostica ampellicida*).

Le protocole est le suivant. Les tests sont réalisés en boîte de Pétri, sur milieu synthétique Avoine-Malt-Agar. Des courbes d'inhibition de croissance sont réalisées en utilisant différentes concentrations des produits afin de déterminer les IC 50 et CMI (concentration inhibant à 50 % et la concentration minimale inhibitrice). Le produit à tester est incorporé au milieu nutritif à raison de 1 mL de produit par boite de Pétri, à raison de 4 boites par dose de produit. L'inoculation des agents pathogènes (extemporanément), est réalisée par le dépôt de trois implants mycéliens par boite de Pétri. Des contrôles sont réalisés en appliquant la même quantité d'eau distillée stérile. La taille de la croissance mycélienne est mesurée après 10 -14 jours d'incubation à 22 °C. L'efficacité du traitement sur le black-rot est déterminée d'après la formule ci-après.

[Math. 1]

$$\% \text{ inhibition} = 100 \times \left( 1 - \frac{\% \text{ croissance "traités"}}{\% \text{ croissance "témoins"}} \right)$$

Les résultats sont présentés dans le tableau 2 ci-après.

[Tableau 2]

| modalités | dilutions | % inhibition | SEM |
|---|---|---|---|
| BC C4 | 0.25 | 100.00 | 0.00 |
| BC C8 | 0.125 | 100.00 | 0.00 |
| BC C20 | 0.05 | 80.00 | 5.28 |
| BC C50 | 0.02 | 58.86 | 1.66 |
| BC C100 | 0.01 | 46.05 | 3.43 |
| BC C500 | 0.002 | 38.41 | 6.75 |

A partir d'une courbe dose-réponse, la CMI, concentration moyenne inhibitrice (concentration minimale pour avoir 100 % d'inhibition de croissance) et l'IC50, concentration induisant une réduction de 50 % de la croissance de l'agent pathogène ont été calculé.

Les résultats induisant 100, 80 et 50 % d'inhibition de croissance du Black-rot dans les tests *in vitro* sont présentés dans le tableau 3 ci-après.

[Tableau 3]

| | x= concentrations BC | x= dilutions de la fraction pu re |
|---|---|---|
| CMI | 0.216 | 5 |
| CI80 | 0.056 | 18 |
| CI50 | 0,007 | 138 |

Ce biocontrôle à base de *B. subtilis* présente une totale efficacité dans ces tests *in vitro,* reflétant un mode d'action direct (antibiose). Cette inhibition totale est obtenue avec une dilution minimale de 5 fois de la solution mère.

Exemple 9 - Essai d'efficacité du biostimulant selon l'exemple 1 et 4 et du biocontrôle selon l'exemple 3.

Cet essai consiste à évaluer l'efficacité de trois biosolutions selon l'invention contre le mildiou de la vigne. Les biosolutions ont été testées en combinaison avec une dose réduite de 30% de cuivre, à savoir le biocontrôle fongicide selon l'exemple 3 (M4), le biostimulant selon l'exemple 1 (M5) et le biostimulant selon l'exemple 4 (M6), comparées avec des modalités témoins non traités (M1), dose pleine de cuivre seule (M2) et dose réduite de cuivre seule (M3).

Plusieurs campagnes de traitement ont été menées et décrites dans le tableau 4 ci-après

[Tableau 4]

| Traitements | Dates | Stades | Dose Cuivre métal g/ha |
|---|---|---|---|
| T1 | 21/04/2023 | 13 | 200 |
| T2 | 28/04/2023 | 53 | 250 |
| T3 | 04/05/2023 | 54 | 250 |
| T4 | 10/05/2023 | 55 | 300 |
| T5 | 17/05/2023 | 56 | 300 |
| T6 | 25/05/2023 | 57 | 300 |
| T7 | 02/06/2023 | 65 | 300 |
| T8 | 08/06/2023 | 71 | 400 |
| Total | | | 2300 |

Les résultats sont présentés en Figure 1.

Les résultats des essais en parcelles de vigne menés d'avril à juillet 2023 démontrent une efficacité supérieure des biosolutions (modalités M4, M5, M6) en comparaison avec la modalité dose pleine de cuivre (M2) et la modalité dose réduite de cuivre seule (M3). En effet, les moyennes des fréquences d'attaque et d'intensité du mildiou sur feuilles des 3 biosolutions sont inférieures aux modalités dose pleine et dose réduite de cuivre. Cela signifie que les 3 biosolutions évaluées (biocontrôle fongicide, biostimulant selon l'exemple 1 et biostimulant selon l'exemple 4 ont la capacité de substituer au moins 30% de la dose de cuivre et d'apporter une efficacité supplémentaire dans la lutte contre le mildiou de la vigne.

**Revendications**

1.  Procédé de transformation de biodéchets et sous-produits issus de l'industrie agroalimentaire en biosolutions destinés à améliorer la fertilisation et/ou la protection des cultures, ou la santé animale, ledit procédé comprenant les étapes suivantes :

    a. collecte des biodéchets,

    b. séparation physique de la fraction solide et de la fraction liquide des biodéchets collectés,

    c. récupération de la fraction liquide,

    d. extraction des molécules d'intérêt présentes dans ladite fraction liquide,

    e. éventuellement, fermentation comprenant l'ajout d'au moins un microorganisme exogène d'intérêt,

    f. stabilisation du produit obtenu à l'étape d'extraction ou de fermentation.

    g. éventuellement, filtration du produit stabilisé.

2.  Procédé de transformation selon la revendication précédente, **caractérisé en ce que** l'étape de séparation est réalisée au moyen d'un décanteur-centrifugeur.

3.  Procédé de transformation selon l'une des revendications précédentes, **caractérisé en ce que** l'extraction des molécules d'intérêt est une extraction par chimie verte.

4.  Procédé de transformation selon la revendication précédente, **caractérisé en ce que** l'étape d'extraction comprend les sous-étapes suivantes :

    • macération à froid avec solvant alcoolique issu de la fermentation naturelle,

    • décoction, et

    • éventuellement, une étape d'extraction sous vide ou par ultrason ou micro-onde.

5.  Procédé de transformation selon l'une des revendications précédentes, **caractérisé en ce que** ledit procédé comprend une étape supplémentaire de décoction de la fraction liquide obtenue après l'étape de récupération et d'extraction.

6.  Procédé de transformation selon l'une des revendications précédentes, **caractérisé en ce que** la fermentation est réalisée à une température comprise entre 17 et 37°C et/ou un pH compris entre 3 et 7, préférentiellement entre 5 et 6.

7.  Procédé de transformation selon l'une des revendications précédentes, **caractérisé en ce que** la stabilisation est réalisée à chaud par traitement thermique ou à froid par filtration et traitement acide.

8.  Procédé de transformation selon la revendication précédente, **caractérisé en ce que** le traitement thermique est une stabilisation à chaud entre 75 et 80°C, ou le traitement acide est un dosage choisi parmi l'acide acétique, peracétique, propionique, et lactique.

9.  Procédé de transformation selon l'une des revendications précédentes, **caractérisé en ce que** le microorganisme exogène d'intérêt est choisi parmi un ferment lactique, une bactérie d'un genre choisi parmi *Bacillus, Lactobacillus, Pseudomonas, Acetobacter, Nitrobacter, Nitrosomonas, Rhizobium, Agrobacterium,* et *Streptomyces ;* un champignon du genre *Trichoderma,* une levure du genre *Saccharomyces,* ou *Aureobasidium* ; et leurs combinaisons.

10. Procédé de transformation selon l'une des revendications précédentes, **caractérisé en ce que** les biodéchets sont choisis parmi les sous-produits brassicoles, les sous-produits cidricoles, les sous-produits vinicoles, les sous-produits oléicoles, les sous-produits de distillerie, les sous-produits du cacao, les sous-produits du café, les sous-produits d'amidonneries, les sous-produits des sucreries, les sous-produits des pressoirs de jus de fruit, et les sous-produits de l'industrie laitière et fromagère, et leurs combinaisons.

**11.** Procédé de transformation selon l'une des revendications précédentes, **caractérisé en ce que** la biosolution est choisie parmi les produits de biocontrôle, les produits de biostimulation, les additifs technologiques, les conservateurs biologiques d'ensilage, les additifs zootechniques, les additifs agronomiques, les biofertilisants, les conservateurs agroalimentaires, les conservateurs ou produits phytosanitaires post-récolte, et les probiotiques.

**12.** Procédé de transformation selon l'une des revendications précédentes, **caractérisé en ce que** la biosolution est un produit de biostimulation ou un biofertilisant et le microorganisme exogène d'intérêt est une bactérie appartenant au genre choisi parmi *Lactobacillus, Bacillus, Trichoderma, Saccharomyces, Acetobacter,* et leurs combinaisons.

**13.** Procédé de transformation selon l'une des revendications précédentes, **caractérisé en ce que** la biosolution est un produit de biocontrôle et le microorganisme est choisi parmi *Bacillus, Trichoderma, Streptomyces, Pseudomonas, Aureobasidium, Saccharomyces,* et leurs combinaisons.

**14.** Procédé de transformation selon l'une des revendications précédentes, **caractérisé en ce que** la biosolution est un additif zootechnique ou un probiotique et le microorganisme exogène d'intérêt est choisi parmi *Bacillus, Lactobacillus, Saccharomyces* et leurs combinaisons.

**15.** Procédé de transformation selon l'une des revendications précédentes, **caractérisé en ce que** la biosolution est un additif agroalimentaire, ou un conservateur d'ensilage, ou un conservateur alimentaire et le microorganisme exogène d'intérêt est choisi parmi *Bacillus, Lactobacillus, Saccharomyces* et leurs combinaisons.

Figure 1